# EUROPÄISCHE PATENTSCHRIFT

(11) **EP 1 261 394 B1**
(45) Veröffentlichungstag und Bekanntmachung des Hinweises auf die Patenterteilung: **07.09.2005**
(21) Anmeldenummer: 01917076.0
(22) Anmeldetag: 07.03.2001
(51) Int. Cl.: A61N 5/10

(54) **IONENSTRAHLANLAGE ZUR BESTRAHLUNG VON TUMORGEWEBEN**
ION BEAM SYSTEM FOR IRRADIATING TUMOUR TISSUES
INSTALLATION A FAISCEAU IONIQUE POUR L'IRRADIATION DE TISSUS TUMORAUX

(30) Priorität: 07.03.2000 DE 10010523
(43) Veröffentlichungstag der Anmeldung: 04.12.2002
(73) Patentinhaber: Gesellschaft für Schwerionenforschung mbH, 64291 Darmstadt (DE)
(72) Erfinder: KRAFT, Gerhard, 64291 Darmstadt (DE)
(74) Vertreter: Boeters, Hans Dietrich
(86) Internationale Anmeldenummer: PCT/EP2001/002575
(87) Internationale Veröffentlichungsnummer: WO 2001/066187

(56) Entgegenhaltungen:
- EP-A- 0 864 337
- EP-A- 0 911 064
- US-A- 4 870 287
- GRAFFMAN S; JUNG B; LARSSON B: "design studies for a 200 MeV proton clinic for radiotherapy" PROCEEDINGS OF THE 6TH INTERNATIONAL CYCLOTRONS CONFERENCE, Nr. 9, 1972, Seiten 603-615, XP001005992
- CHU W T ET AL: "INSTRUMENTATION FOR TREATMENT OF CANCER USING PROTON AND LIGHT-ION BEAMS" REVIEW OF SCIENTIFIC INSTRUMENTS,AMERICAN INSTITUTE OF PHYSICS. NEW YORK,US, Bd. 64, Nr. 8, 1. August 1993 (1993-08-01), Seiten 2055-2122, XP000393868 ISSN: 0034-6748
- PEDRONI E: "BEAM DELIVERY" HADRONTHERAPY IN ONCOLOGY: PROCEEDINGS OF THE INTERNATIONAL SYMPOSIUM ON HADRONTHERAPY,XX,XX, 1994, Seiten 434-452, XP000869931 in der Anmeldung erwähnt

## Beschreibung

Die Erfindung betrifft eine Ionenstrahlanlage zur Bestrahlung von Tumorgeweben gemäß dem Oberbegriff des unabhängigen Anspuchs.

EP-A-0 864 337 offenbart eine Ionenstrahlanlage zur Bestrahlung von Tumorgewebe.

Aus US 4,870,287 ist eine Protonenstrahlanlage zur selektiven Erzeugung und zum Transport von Protonenstrahlen von einer einzelnen Protonenquelle über einen Beschleuniger zu einer Mehrzahl von Patientenbehandlungsstationen bekannt, wobei jede der Behandlungsstationen ein drehbares Trommelgestell, das im folgenden Gantry genannt wird, aufweist. Dieses Gantry liefert den Protonenstrahl in der bekannten Anlage unter unterschiedlichen Bestrahlungswinkeln zu einem Patienten, der auf einer Patientenliege fixiert ausgerichtet ist. Eine Zusammenstellung von Gantrysystemen ist von Pedroni bekannt, aus "Beam Delivery" in Hadron Therapy in Oncology, Herausgeber U.A. Maldi und B. Larsson, Elsevier 1994, Seiten 434 - 452.

Solange derartige Ionenstrahlanlagen zur Bestrahlung von Tumorgeweben mit dem leichtesten Ion des Periodensystems, nämlich dem Wasserstoffion oder Proton, arbeiten, sind die Umlenkmagnete für ein Gantry und seine Massen relativ klein und beherrschbar. Sollen jedoch schwerere Ionen wie das Ion des Kohlenstoffs oder andere eingesetzt werden, so müssen um ein Mehrfaches größere Ablenkmagnete eingesetzt werden, um die hochbeschleunigten Ionen von der Achse eines Gantry auf den Umfang eines Gantry und zurück zum Zentrum des Gantry, in dem der Patient positioniert ist, zu lenken. Gleichzeitig müssen entsprechend große Massen als Gegengewicht zu den Ablenkmagneten in einem Gantry vorgesehen werden, so daß das zu drehende und auf wenige Millimeter genau zu beherrschende Drehgestell eines Gantry mehrere hundert Tonnen wiegt. Mit zunehmender Ionenmassenzahl wird somit die propagierte Gantrylösung schwerer, unhandlicher und erfordert immer größere Gebäude zur Unterbringung der Behandlungsanlagen.

Ziel jeder Strahlentherapie ist es, eine möglichst hohe Strahlendosis in einem engumgrenzten Gebiet, dem Tumorvolumen, zu deponieren und das umliegende gesunde Gewebe maximal zu schonen. In der herkömmlichen Röntgenstrahltherapie kann wegen des exponentiellen Abfalls der Photonendosis mit der Eindringtiefe in der konventionellen subcutanen Strahlentherapie von tieferliegenden Tumoren eine hohe Herddosis nur dadurch erreicht werden, daß in einer kreuzenden Bestrahlungstechnik der Strahl von mehreren Richtungen auf das Tumorvolumen gelenkt wird. Dadurch wird die Belastung des gesunden Gewebes vor und hinter dem Tumorvolumen verringert. In der klinischen Praxis sind zwei bis drei Eingangswinkel üblich und mit einer inversen Dosisplanung bei intensitätsmodulierter Therapie mit Photonen werden oft bis zu neun oder zehn Eingangskanäle, d.h. Bestrahlungswinkelstellungen, geplant. Diese Mehrfeldbestrahlungen sind insbesondere mit den bekannten Gantrysystemen durchführbar.

In analoger Weise zu dieser Photonenstrahltherapie sind auch in der Ionenstrahltherapie mehrere Eingangspforten wünschenswert, obwohl durch das invertierte Dosisprofil der Ionenstrahlen die Dosis im Eingangskanal kleiner ist als die Dosis im Bereich des Tumorvolumens. Jedoch würde die Verteilung der nicht vermeidbaren Eingangsdosis auf mehrere Bestrahlungswinkel auch bei der Ionenstrahltherapie einen weiteren klinischen Vorteil bedeuten. Deshalb werden die bekannten Ionenstrahlanlagen für eine Protonenstrahlbehandlung mit einer Bestrahlungsapplikation aus allen Richtungen durch entsprechende Gantrysysteme ausgestattet.

Insoweit sind die aus der Patentschrift US-PS 4,870,287 bekannten Gantrysysteme den Gantrysystemen aus der Photonen- bzw. Röntgentherapie angepaßt. Sie lenken den Strahl zunächst aus der Patientenachse ab und biegen ihn dann unter 90° auf den Patienten zurück. Variable Bestrahlungswinkel werden dann dadurch erreicht, daß das ganze Ablenksystem längs der Strahlrichtung um 360° mit Hilfe des Gantry gedreht wird. Die mechanische Drehung dient vor allen Dingen dazu, die Einstellung der Magnete nicht zu variieren und nur eine mechanische Drehung ausführen zu müssen. Dieser Vorteil einer einfachen mechanischen Drehung ohne Variation der Einstellung der Elektromagnete gilt jedoch nur solange, wie das Gantrysystem für die Behandlung einen divergenten Ionenstrahl anwendet. Bei der Implementierung eines konzentrierten bleistiftdünnen Ionenstrahls unter Einsatz eines aktiven Scansystems zum Abtasten des Tumorvolumens ist jedoch die Konstanz und Invariabilität des Magnetfeldes nicht mehr gegeben, da die Strahlenergie und damit die magnetische Steifigkeit gemäß den erforderlichen Energieschritten, die für diesen Bleistiftstrahl anzuwenden sind, verwendet werden müssen. Damit entfällt der Hauptgrund für ein Gantry mit festen Magneteinstellungen, zumal bereits für das Scansystem der Bleistiftstrahl orthogonal in X- und Y-Richtung gegenüberdem zentralen Ionenstrahlablenkbereich abzulenken ist.

Darüber hinaus zeigt die Erfahrung mit dem aus der PatentschriftUS-PS 4,870,287 bekannten Protonenstrahltherapiesystem, daß bei der Ionenstrahltherapie von tiefliegenden Tumoren nicht alle möglichen Einstrahlungswinkel eines Gantry mit gleicher Häufigkeit eingesetzt werden. Vielmehr hat es sich gezeigt, daß es einen großen Bereich selten genutzter Bestrahlungswinkelzonen gibt, da häufig auftretende Tumorarten häufig wiederkehrende eingeschränkte Winkeleinstellungen des Gantry erfordern. Insoweit hat es sich gezeigt, daß herkömmliche Gantrysysteme für die Ionenstrahltherapie keine optimalen Lösungen darstellen, da ein großer Anteil der möglichen Bestrahlungswinkel eines Gantry wenig genutzt bleibt.

Charakteristisch für die bekannten und geplanten Ionentherapiesysteme ist, daß der Teilchenstrahl unter festem Winkel durch das Gantrysystem geleitet wird und daß die Variation des Winkels nur mechanisch durch Drehung des Gesamtsystems erreicht werden kann. Aufgrund der hohen Energie der Teilchen von 200 MeV für Protonen und ca. 400 MeV/U für Kohlenstoffionen und aufgrund der für das Scannen der Ionenstrahlen notwendigen großflächigen Aperturen sind Ablenkmagnete von hoher Magnetfeldstärke mit großflächigen Aperturen erforderlich. Das bedeutet, daß die Elektromagnete ein erhebliches Ausmaß und Gewicht erreichen. Ein tonnenförmiges Gantry für Kohlenstoffionen ist deshalb für einen Radius von 7 m und eine Länge von 15 - 20 m und einem Gewicht von 300 - 400 t konzipiert, wobei ca. 50 t allein auf ein Betongewicht als Gegengewicht zu den Magneten entfällt. Bei diesen erheblichen Gewichten gewinnt die Toleranz der Lager und damit die Positionsgenauigkeit des Strahls eine zunehmende Bedeutung. Beim Patienten liegen die Toleranzgrenzen jedoch im Millimeterbereich. Derartige Toleranzgrenzen sind mit den bisher gebauten Gantrysystemen schwer einzuhalten.

Beim Bau von Ionenstrahlanlagen für Kliniken ist der Bau von mehreren Gantrysystemen ein wesentlicher Kostenfaktor. Diese Kosten beziehen sich auf das eigentliche Gantry mit mehr als 15 Millionen DM pro System, wie dem Bau geeigneter Betriebshallen und mehr als 14 m Höhe und Breite und über 20 m Länge, das bedeutet einen umbauten Raum von mehr als 4.000 m³. Diese Räume sind dickwandig mit Beton abzuschirmen. Außerdem stellt der geplante Einsatz von Ionenrasterverfahren an die notwendige mechanische Präzision bisher noch ein ungelöstes Problem dar. Deshalb muß beim Ionenrasterverfahren die erforderliche Präzision von 1 mm nach jeder Einstellung und für jede Neubehandlung kontrolliert, verifiziert und korrigiert werden.

Aufgabe der Erfindung ist es, die Nachteile im Stand der Technik für Ionenstrahlanlage zu überwinden und Bestrahlungssysteme anzugeben, die den Bedürfnissen nach Kostenminderung und Raumersparnis bei gleichzeitig erhöhter Präzision entgegenkommen.

Diese Aufgabe wird mit den Merkmalen des Gegenstands des unabhängigen Anspruchs gelöst. Bevorzugte Weiterbildungen der Erfindung ergeben sich aus den Merkmalen der abhängigen Ansprüche.

Die Lösung dieser Aufgabe geht davon aus, daß der Patient in liegender Haltung auf einer Patientenliegen fixiert ist und diese horizontale Lage vor und während der Bestrahlung nicht verändert wird. Diese horizontale Lage auf einer Patientenliege hat den Vorteil, daß unkontrollierte Organbewegungen vermieden werden, wie sie nachteilig bei einer sitzenden Position oder einer Drehung während der Behandlung auftreten. Das bedeutet, daß die vorgesehene Drehvorrichtung der Patientenliegen lediglich zum Einstellen des Bestrahlungswinkels eingesetzt wird, nicht aber während der aktiven Bestrahlung betätigt wird. Ein Bestrahlungswinkel des liegenden Patienten kann sich somit in vorteilhafter Weise aus allen möglichen Winkeln (4π) zusammensetzen, indem durch die Drehvorrichtung der Patientenliege eine 360°-Positionierung (2π) der horizontalen Patientenliege auf der Bestrahlungsebene möglich ist und zusätzlich eine Positionierung der Strahlrichtung um 180° (π) orthogonal dazu, d.h. eine Bestrahlung kann von senkrecht oben und seitlich um den Patienten nach senkrecht unten durch die erfindungsgemäßen Bestrahlungssysteme erfolgen. Somit kann bei der erfindungsgemäßen Lösung vorteilhaft auf ein 360° drehendes Gantry verzichtet werden und ist aufgrund der Aufgabenstellung auch nicht wünschenswert, da ein um 360° drehendes Gantry den Platz um den Patienten einschränkt und somit den Zugang sowie die Installation von Diagnoseeinheiten, wie beispielsweise einer PET-Kamera, behindert.

Dazu weist die erfindungsgemäße Lösung neben einer Drehvorrichtung der Patientenliege um eine vertikale Achse mindestens eines der folgenden Bestrahlungssysteme auf: ein erstes Bestrahlungssystem mit einem asymmetrischen, örtlich feststehenden Scansystem, das einen zentralen Ionenstrahlablenkbereich für Ablenkwinkel von bis zu ±15° gegenüber der horizontalen Richtung aufweist und eine Abtastung eines Tumorvolumens in dem zentralen Ionenstrahlablenkbereich ermöglicht und eine zusätzliche Hubvorrichtung für die Patientenliege aufweist, und ein zweites Bestrahlungssystem, das eine Ionenstrahlablenkvorrichtung für eine gegenüber dem ersten Bestrahlungssystem größeren Ablenkwinkelbereich und eine stromabwärts der Ionenstrahlablenkvorrichtung angeordnete und synchron mit dem Ablenkwinkel der Ionenstrahlablenkvorrichtung schwenkbare symmetrische Scaneinheit zur Abtastung des Tumorvolumens aufweist.

Vorzugsweise weist die Ionenstrahlanlage mindestens zwei erste Bestrahlungssysteme und ein zweites Bestrahlungssystem auf in jeweils getrennten Bestrahlungsräumen. Diese bevorzugte Aufteilung auf zwei Bestrahlungssystem mit eingeschränktem Ablenkwinkel bis zu ±15° berücksichtigt die Tatsache, daß zwei Drittel der Tumorgewebebehandlungen mit einer derartigen Ionenstrahlanlage abgedeckt werden können und somit in diesen Bestrahlungssystemen mit eingeschränktem Ablenkwinkel auf ±15° ein asymmetrisches Scansystem, das in X-Richtung die Breite des Tumorvolumens abdeckt und in Y-Richtung nicht nur die Tiefe des Tumorvolumens berücksichtigt, sondern durch Vergrößerung des Ablenkmagnetsystems in Y-Richtung den zentralen Ionenstrahlablenkbereich auf ±15° erweitert und gleichzeitig im Bereich des zentralen Ionenstrahlablenkbereichs ein Scannen über die Tiefe des Tumorvolumens ermöglicht. Dazu wird die Patientenliege zusätzlich zur Drehmöglichkeit mit einer Hubmöglichkeit versehen, um in dem Ablenkwinkel bzw. dem Bestrahlungswinkel des zentralen Ionenstrahlablenkbereichs positioniert zu werden. Der verbleibende Anteil an Patienten von etwa 1 Drittel, für die größere zentrale Ionenstrahlablenkbereiche erforderlich werden, wird mit einem zweiten Bestrahlungssystem behandelt, das unabhängig von einer Scaneinheit zunächst mit Hilfe äußerer Ablenkmagneteinheiten den Ionenstrahl bis zu ± 90° gegenüber der horizontalen Ebene ablenken kann. Mit dem Ablenkwinkel der Ionenstrahlablenkvorrichtung dieses zweiten Bestrahlungssystems wird synchron eine symmetrische Scaneinheit zur Abtastung des Tumorvolumens in die Richtung des Ablenkwinkels geschwenkt, so daß der Ablenkmagnet für derart große Ablenkwinkel und seine Apertur kleingehalten werden können und ebenso die symmetrische Scaneinheit nur zum Ablenken und Abtasten des Tumorvolumens auszulegen ist, was eine erhebliche Gewichts- und Volumenersparnis gegenüber den bisher vorgeschlagenen Gantrysystemen bedeutet.

Da die Vorbereitung und Fixierung des Patienten auf der Patientenliege die größte Zeit des gesamten Bestrahlungszyklus in einer Ionenstrahlanlage zur Bestrahlung von Tumorgeweben einnimmt, weist die Ionenstrahlanlage mindestens einen Vorbereitungsraum pro Bestrahlungssystem mit jeweils einer Patientenliege auf. Bevorzugt sind zwei und mehr Vorbereitungs- und Nachsorgeräume pro Bestrahlungssystem einzusetzen. Die Anzahl der erforderlichen Bestrahlungsräume steigt somit mit der Anzahl der eingesetzten Bestrahlungssysteme, und die Anzahl der Vorbereitungsräume steigt mit dem Zeitaufwand für das Fixieren eines Patienten auf der horizontalen Patientenliege im Verhältnis zum eigentlichen Bestrahlungsvorgang im Bestrahlungsraum.

Während für einen Bestrahlungswinkel zwischen -15° und +15° lediglich eine asymmetrische Scaneinheit des ersten Bestrahlungssystems erforderlich ist und außer einer Hub- und einer Dreheinstellung der Patientenliege nur der Erregerstrom für einen der Ablenkmagnete des Scansystems bereitzustellen ist und ein erweitertes Austrittsfenster für den Ionenstrahl aus dem asymmetrischen Scansystem vorzusehen ist, wird in einer bevorzugten Ausführungsform der Erfindung für das zweite Bestrahlungssystem eine mit variablem Ablenkwinkel feststehende Ionenstrahlablenkvorrichtung eingesetzt, die seitlich von der Patientenliege angeordnet ist, wobei die Patientenliege eine zusätzliche Hubvorrichtung aufweist, die wesentlich größere Höhenunterschiede überwindet als die Hubvorrichtung der Patientenliege des ersten Bestrahlungssystems. Der für diese größeren Bestrahlungswinkel erforderliche Ablenkmagnet kann jedoch mit einer kleineren Apertur ausgestattet sein, da der bleistiftförmige Ionenstrahl den Umlenkmagneten passiert ohne eine Vorablenkung durch eine Scaneinheit in X-Richtung zu erfahren. Erst nach der Umlenkung wird dann eine entsprechend dimensionierte symmetrische Scaneinheit angeschlossen, die mit dem Umlenkwinkel schwenkbar ist, so daß wiederum das gesamte Tumorvolumen im zentralen Ionenstrahlablenkbereich abgetastet werden kann.

Alternativ zum seitlich von der Patientenliege angeordneten Ablenkmagneten für den Ionenstrahl kann in einer weiteren Ausführungsform der Erfindung dieser Magnet auch vertikal über dem Patienten angeordnet sein und die Patientenliege nicht mit einer Hubvorrichtung, sondern mit einer lateral in der horizontalen Ebene verschiebbaren Vorrichtung ausgestattet sein. Auch mit dieser bevorzugten alternativen Ausführungsform der Erfindung kann erreicht werden, daß ohne rotierendes Gantry ein großer Strahlungswinkelbereich für die Behandlung von Tumoren zur Verfügung steht.

In einer weiteren bevorzugten Ausführungsform der Erfindung weist das zweite Bestrahlungssystem eine auf einer horizontalen linearen Bahn verschiebbare Ionenstrahlablenkvorrichtung mit variablem Ablenkwinkel auf, und die Patientenliege ist lateral in allen Richtungen fixiert. Das heißt, daß die Patientenliege weder eine Hubvorrichtung noch eine Verschiebemöglichkeit in den beiden Achsen der horizontalen Ebene aufweist. Lediglich eine Drehung um die vertikale Achse ist möglich, um die Patientenliege in einem entsprechenden Drehwinkel zur Bestrahlungsrichtung zu fixieren. Die horizontale lineare Bahn, auf der verschiebbar die Ionenstrahlablenkvorrichtung gleitet, kann oberhalb oder unterhalb des Patienten angeordnet sein, wobei mit dem Verschieben gleichzeitig der Erregerstrom in dem Magneten variiert, um den Ionenstrahl in unterschiedlichen Winkeln in Richtung auf den fixierten Patienten abzulenken.

In einer weiteren bevorzugten Ausführungsform der Erfindung weist das zweite Bestrahlungssystem eine mit variablem Ablenkwinkel feststehende Ionenstrahlablenkvorrichtung und eine auf einer vertikalen linearen Bahn verschiebbare Ionenstrahlablenkvorrichtung mit variablem Ablenkwinkel auf. Die beiden variablen Ablenkwinkel sind derart aufeinander abgestimmt, daß die Patientenliege lateral fixiert bleiben kann und trotz unterschiedlichem Bestrahlungswinkel keine laterale Verschiebung der Patientenliege erforderlich ist. Die Scaneinheit ist der auf einer vertikalen linearen Bahn verschiebbaren Ionenstrahlablenkvorrichtung nachgeordnet und kann folglich vollständig symmetrisch ausgebildet sein, um das Tumorgewebe abzutasten. Diese Lösung hat den Vorteil gegenüber einer horizontalen Verschieblichkeit der Ionenstrahlablenkvorrichtung, daß für die vertikale lineare Bahn der verschiebbaren Ionenstrahlablenkvorrichtung lediglich der Ablenkmagnet mit einer Hubvorrichtung auszustatten ist. Während also der Patient in seiner festgelegten Stellung ohne laterale Verschiebung verharrt, wird in diesem Fall zur Positionierung des Ionenstrahls ein zweiter Ablenkmagnet mit einer Hubvorrichtung herauf- und heruntergefahren, um den Bestrahlungswinkel zu variieren. Ist der Bestrahlungswinkel einmal eingestellt, so bleibt er während der Bestrahlung unverändert und konstant. Auch der Patient wird während der Bestrahlung nicht mehr bewegt.

Bei einer weiteren bevorzugten Ausführungsform der Erfindung weist das zweite Bestrahlungssystem eine auf einer schiefen linearen Bahn gegenüber der horizontalen Richtung unter einem Winkel α linear verschiebbare Ionenstrahlablenkvorrichtung für einen Ablenkwinkelbereich von -α bis -(α + 90°) auf, während die Patientenliege lateral fixiert bleibt. Dazu wird der horizontal in den Behandlungsraum eintretende Ionenstrahl zunächst unter einem Winkel α in Richtung auf die linear verschiebbare Ionenstrahlablenkvorrichtung abgelenkt und diese Ionenstrahlablenkvorrichtung, die nur noch einen linearen Verschiebemechanismus benötigt, lenkt den Ionenstrahl in einem vorbestimmten Winkel auf den Patienten. Dieser Winkel kann von 0 bis 90° reichen und in einer entsprechend alternativen Konstruktion auch von 0 bis -90° ausgelegt werden. In dem ersten Fall wird die schiefe lineare Bahn von seitlich neben dem Patienten nach oberhalb des Patienten ausgerichtet und in dem anderen Fall wird die schiefe lineare Bahn, auf die die Ionenablenkvorrichtung gleitet, von einer Position seitlich vom Patienten zu einer Position unterhalb des Patienten verschoben. Mit einem derartigen zweiten Bestrahlungssystem und linear verschiebbarer Ionenstrahlablenkvorrichtung wird die aufwendige Konstruktion eines Gantry entbehrlich und im wesentlichen folgende Vorteile erreicht:
1. Eine höhere mechanische Stabilität aufgrund der linearen Bewegung auf einer schiefen Ebene verglichen mit einer Rotation eines Gantry,
2. Wegfall von sonst erforderlichen Ausgleichsgewichten,
3. vergleichsweise geringeres Gewicht aller beweglichen Teile,
4. vergleichsweise leichtere Dipolmagnete, da nur kleine Aperturen notwendig werden,
5. eine Verringerung des umbauten abgeschirmten Bestrahlungsraumes auf etwa 600 cm³, d.h. weniger als ein Viertel des Raumes eines Rotationsgantry, und
6. eine geringere Einschränkung des Raumes um die Patientenliege herum und damit eine verbesserte Möglichkeit der Aufstellung anderer Überwachungssystems wie PET (Photonen-Emissions-Topographiegerät).

Die gleichen Vorteile können erreicht werden, wenn in einer weiteren bevorzugten Ausführungsform der Erfindung das zweite Bestrahlungssystem eine mit variablem Ablenkwinkel feststehende Ionenstrahlablenkvorrichtung aufweist und zusätzlich eine auf einer schiefen linearen Bahn gegenüber der horizontalen Richtung unter einem Winkel α linear verschiebbare Ionenstrahlvorrichtung für einen Ablenkwinkelbereich von -α bis -(α + 90°) aufweist, wobei die Patientenliege lateral fixiert ist. In dieser Ausführungsform ist vorgesehen, daß der Ionenstrahl oberhalb des Patienten in den Bestrahlungsraum eintritt und von einer feststehenden Ionenstrahlablenkvorrichtung in unterschiedliche Ablenkwinkel abgelenkt wird, und diese wiederum durch die zweite auf einer schiefen linearen Bahn verschiebbare Ionenstrahlablenkvorrichtung den Ionenstrahl zum Patienten hin ablenkt. Auch in dieser Ausführungsform sind die Aperturen klein, da eine symmetrische Scaneinheit der verschiebbaren Ionenstrahlablenkvorrichtung nachgeordnet ist und ihrerseits schwenkbar dem Ablenkwinkel des Ionenstrahles folgt.

In einer bevorzugten Ausführungsform der Erfindung ist der Winkel α der schiefen linearen Bahn 45°. Mit dieser schiefen Bahn um 45° wird erreicht, daß der Patient alle Bestrahlungswinkel im Quadranten eines Kreises von seitlich vom Patienten bis vertikal zum Patienten erreichen kann, ohne daß die Patientenliege zu verstellen ist.

Zur Umlenkung des Ionenstrahls aus der horizontalen Richtung in eine schiefe Bahn weist vorzugsweise das zweite Bestrahlungssystem einen ersten Umlenkmagneten für den hochbeschleunigten horizontalen Ionenstrahl auf. Ein zweiter Umlenkmagnet, der zur verschiebbaren Ionenstrahlablenkvorrichtung gehört, lenkt vorzugsweise den Strahl aus der schiefen linearen Bahn in eine horizontale Richtung und ein nachgeschaltetes geeignetes magnetisches Ablenksystem oder ein Solenoidmagnet bewirkt eine weitere Umlenkung zwischen 0 und 90° unter gleichzeitiger Bewegung der gesamten Ionenstrahlablenkvorrichtung je nach Einstellung des Erregerstroms des geeigneten magnetischen Ablenksystems oder des Solenoidmagneten.

Eine bevorzugte Ausführungsform des ersten Bestrahlungssystems sieht einen ersten Ablenkmagneten zum Abtasten des Tumorvolumens in einer X-Richtung der orthogonalen Ebene zum zentralen Ionenstrahlablenkbereich vor und einen Ablenkmagneten zum Ablenken in die Richtung des zentralen Ionenstrahlablenkbereichs in einer Y-Richtung der orthogonalen Ebene zum zentralen Ionenstrahlablenkbereich mit zusätzlicher Überlagerung zum Abtasten des Tumorvolumens in dieser Richtung.

Diese Ausführungsform des ersten Bestrahlungssystems hat den Vorteil, daß keinerlei zusätzliche Ablenkmagnete erforderlich sind und der größte Teil der Patienten mit Tumoren in einer derartigen Ausführungsform des ersten Bestrahlungssystems mit eingeschränktem Ablenkwinkel behandelt werden kann.

Vorzugsweise beträgt der Abstand zwischen dem Ablenkmagneten in Y-Richtung beim ersten Bestrahlungssystem und der Patientenliege zwischen 5 und 7 m. Andererseits besteht vorzugsweise ein Abstand zwischen der verschiebbaren linearen Bahn des Solenoidmagneten des zweiten Bestrahlungssystems und der Patientenliege von 3 bis 6 m, wobei die mitverschiebbare symmetrische Scaneinheit in diesem Bereich zwischen 3 und 6 m angeordnet ist.

Sofern eine Hubvorrichtung der Patientenliege in einer der Ausführungsformen der Erfindung erforderlich wird, kann mit der Patientenliege gleichzeitig auch der gesamte Boden des Bestrahlungsraumes angehoben werden, so daß der Eindruck für den Patienten, in einem gewöhnlichen Bestrahlungsraum fixiert zu sein, in vorteilhafter Weise bestehen bleibt, und Bedienungspersonal jederzeit vor und nach dem Bestrahlen den Patienten in normaler Arbeitsposition behandeln kann.

Zur Ortsmessung und Überwachung des abtastenden Ionenstrahls weist vorzugsweise die Ionenstrahlanlage in jeder Bestrahlungskammer Ionisationskammermeßvorrichtungen und Vieldrahtkammermeßvorrichtungen auf. Diese Meßvorrichtungen zeigen in vorteilhafter Weise sowohl die Intensität eines Ionenstrahls als auch den Ort eines Ionenstrahls an.

Die gesamte Ionenstrahlanlage ist derart konzipiert, daß in einer bevorzugten Ausführungsform die Bestrahlungssysteme in Bestrahlungsräumen angeordnet sind, die ihrerseits kompakt und kreissegmentförmige nebeneinander liegen. Dieses hat den Vorteil, daß kurze Ionenstrahlwege mit wenigen Ablenkmagneten mit dem Beschleuniger als Ionenstrahlquelle zu verbinden sind. Außerdem ermöglicht diese Anordnung jederzeit in vorteilhafter Weise eine Systemerweiterung, falls dies später erforderlich wird. Somit ergibt sich als optimale Lösung eine Art radialer Aufbau der gesamten Ionenstrahlanlage mit mehreren Bestrahlungsräumen.

In einer weiteren bevorzugten Ausführungsform der Erfindung ist die Ionenstrahlanlage mit einer Sichtkontrolle für die Patientenliege im Bestrahlungsraum ausgestattet, um definierte Drehwinkeländerungen zwischen unterschiedlichen Einstellungen für eine Mehrfeldbestrahlung zu überwachen. Diese Sichtkontrolle ermöglicht vorteilhaft, nach dem Transfer zum Bestrahlungsplatz die Koordinaten des Patientensystems mit denen des Strahlungsraumes zu vergleichen und zu kontrollieren. Eine eventuell nötige Drehung der Patientenliege bei der Mehrfeldbestrahlung aus unterschiedlichen Bestrahlungswinkeln kann durch die Sichtkontrolle von außen gesteuert werden.

In einer weiteren bevorzugten Ausführungsform der Erfindung weist das Bestrahlungssystem Einrastvorrichtungen zur Aufnahme und Positionierung einer Patientenliege im Bestrahlungsraum auf. Derartige Einrastvorrichtungen sind mechanisch komplexe Einrichtungen, die sicherstellen, daß millimetergenau die Patientenliege in Relation zu der Bestrahlungsrichtung und dem Bestrahlungswinkel einstellbar ist. Die Einrastvorrichtung stellt darüber hinaus sicher, daß eine vollständige Kopplung zwischen Aufnahme- und Positioniereinrichtungen für die Patientenliege gewährleistet ist, so daß der Ionenstrahl in dem Bestrahlungsraum exakt das Tumorgewebe abscannen kann.

Weitere Vorteile, Merkmale und Anwendungsmöglichkeiten der Erfindung werden nun anhand von Ausführungsbeispielen unter Bezugnahme auf die beigefügten Zeichnungen näher erläutert.
Fig. 1 zeigt eine Prinzipskizze einer Ionenstrahlanlage zur Bestrahlung von Tumorgeweben gemäß einer Ausführungsform der vorliegenden Erfindung,
Fig. 2 zeigt eine Prinzipskizze eines ersten Bestrahlungssystems mit asymmetrischem, örtlich feststehendem Scansystem,
Fig. 3 zeigt eine Prinzipskizze einer Ausführungsform eines zweiten Bestrahlungssystems mit ortsfester Ionenstrahlablenkvorrichtung und vertikaler Hubvorrichtung einer Patientenliege,
Fig. 4 zeigt eine Prinzipskizze einer weiteren Ausführungsform eines zweiten Bestrahlungssystems mit ortsfester Ionenstrahlablenkvorrichtung und horizontal verschiebbarer Patientenliege,
Fig. 5 zeigt eine Prinzipskizze einer weiteren Ausführungsform eines zweiten Bestrahlungssystems mit ortsfester drehbarer Patientenliege und einer auf einer horizontalen linearen Bahn verschiebbaren Ionenstrahlablenkvorrichtung,
Fig. 6 zeigt eine Prinzipskizze einer weiteren Ausführungsform eines zweiten Bestrahlungssystems mit ortsfester drehbarer Patientenliege und einer auf einer vertikalen linearen Bahn verschiebbaren Ionenstrahlablenkvorrichtung,
Fig. 7 zeigt eine Prinzipskizze einer weiteren Ausführungsform eines zweiten Bestrahlungssystems mit ortsfester drehbarer Patientenliege und einer verschiebbaren Ionenstrahlablenkvorrichtung auf einer schiefen linearen Bahn,
Fig. 8 zeigt eine Prinzipskizze einer weiteren Ausführungsform eines zweiten Bestrahlungssystems mit ortsfester drehbarer Patientenliege und einer Kombination aus ortsfester und auf einer schiefen linearen Bahn verschiebbarer Ionenstrahlablenkvorrichtung,
Fig. 9 zeigt eine Prinzipskizze einer weiteren Ausführungsform eines zweiten Bestrahlungssystems mit ortsfester drehbarer Patientenliege und einer unter einem Winkel von 45° gegenüber der horizontalen Richtung geneigten schiefen linearen Bahn für eine verschiebbare Ionenstrahlablenkvorrichtung,
Fig. 10 zeigt eine Prinzipskizze der in Fig. 9 gezeigten Ausführungsform in zwei weiteren Positionen der verschiebbaren Ionenstrahlablenkvorrichtung.

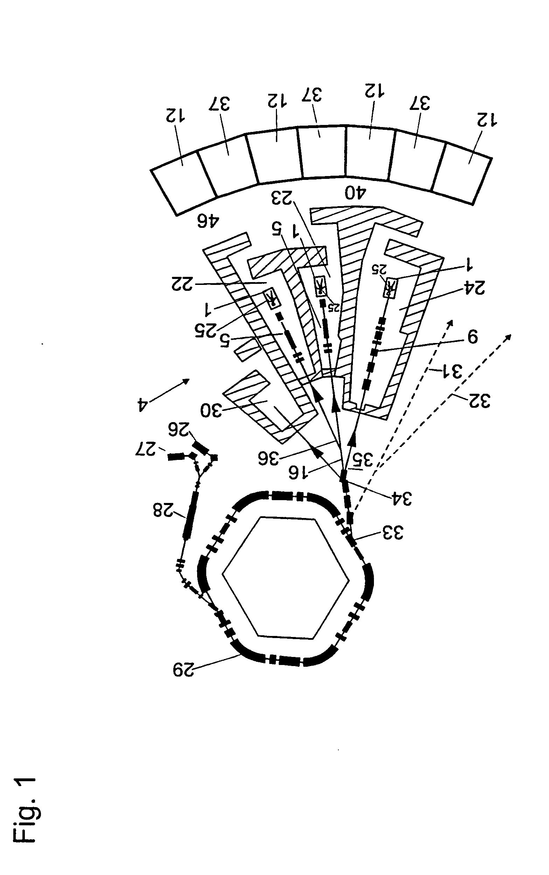

Fig. 1 zeigt eine Prinzipskizze einer Ionenstrahlanlage 4 zur Bestrahlung von Tumorgeweben eines Patienten 25 auf einer Patientenliege 1 gemäß einer Ausführungsform der Erfindung. Eine derartige Ionenstrahlanlage weist unterschiedliche Ionenquellen 26, 27 auf, die unterschiedliche Gase ionisieren, Ionen in einem Massenspektrometer selektieren und die selektierten Ionen zunächst in einer linearen Beschleunigungskammer 28 beschleunigen. Die Ionen werden dann in ein Synchrotron 29 zur weiteren Hochbeschleunigung eingeleitet und nach entsprechender Energieaufnahme als Ionenstrahl 16 aus dem Synchrotron 29 ausgekoppelt. Der Ionenstrahl 16 kann in kreissegmentförmig angeordnete Bestrahlungsräume 22, 23, 24 und 30 abgelenkt werden. In den Bestrahlungsräumen 22, 23 und 24 werden zur Bestrahlung von Tumorgeweben unter unterschiedlichen Bestrahlungswinkeln in bezug auf eine horizontal angeordnete Patientenliege 1 Tumorgewebe mit Ionenstrahlen behandelt. Dazu liefern die Ionenquellen 26 und 27 Ionen wie Kohlenstoffionen, die beim Durchgang durch Gewebe, insbesondere im Abbremsbereich der Ionenbahn im menschlichen Körper besonders wirkungsvoll Tumorgewebe zerstören können.

Die Ionenstrahlanlage dieser Ausführungsform weist eine Patientenliege 1 mit einer Drehvorrichtung um eine vertikale Achse auf und zwei Bestrahlungsräume 22 und 23 mit einem ersten Bestrahlungssystem, das ein asymmetrisches, örtlich feststehendes Scansystem aufweist. Dieses Scansystem kann den horizontalen Ionenstrahl in einen zentralen Ionenstrahlablenkbereich für Ablenkwinkel von bis zu ±15° gegenüber der horizontalen Richtung ablenken. Gleichzeitig kann das asymmetrische Scansystem eine Abtastung des Tumorvolumens in dem zentralen Ionenstrahlablenkbereich durchführen, wozu mittels einer zusätzlichen Hubvorrichtung für die Patientenliege der Patient mit dem Tumorgewebe in den Wirkungsbereich des zentralen Ionenstrahlablenkbereichs gehoben wird.

Während die beiden Bestrahlungsräume 22 und 23 mit einem derartigen asymmetrischen Scansystem und einer Hubvorrichtung für die Patientenliege 1, die gleichzeitig um ihre vertikale Achse gedreht werden kann, ausgestattet sind, ist der in dieser Ausführungsform dritte Bestrahlungsraum 24 mit einem zweiten Bestrahlungssystem ausgestattet, das eine Ionenstrahlablenkvorrichtung für einen gegenüber dem ersten Bestrahlungssystem der Bestrahlungsräume 22 und 23 größeren Ablenkwinkelbereich und eine stromabwärts der Ionenstrahlablenkvorrichtung angeordnete und synchron mit dem Ablenkwinkel der Ionenstrahlablenkvorrichtung schwenkbare symmetrische Scaneinheit zur Abtastung des Tumorvolumens aufweist.

Der Bestrahlungsraum 30 ist Test- und Versuchsraum, in dem insbesondere Überwachungs- und Meßgeräte geprüft und eingestellt werden können. Ferner zeigt Fig. 1 Erweiterungsmöglichkeiten der Ionenstrahlanlage mit den gestrichelten Ionenstrahlrichtungen 31 und 32 und Vorbereitungsräume 12, in denen die Patienten für die Bestrahlungsbehandlung vorbereitet werden können. Diese Ionenstrahlanlage ist aus zwei Bestrahlungssystemen zu einer Bestrahlungseinheit integriert worden und basiert auf den Erfahrungen mit der bekannten Protonengantry-Anlage des US-Patents 4,870,287, die weltweit bisher die einzige Anlage ist, bei der mit einem hohen Patientenaufkommen eine klinische Teilchentherapie durchgeführt wird. Mit der bekannten Anlage werden etwa 1000 Patienten pro Jahr bestrahlt, von denen zwei Drittel bis drei Viertel nur in einem Bestrahlungswinkelbereich von zwei Seiten unter jeweils 0 bis 15° gegenüber einer horizontalen Patientenliege 1 bestrahlt werden. Der restliche Rotationsbereich der bekannten Protonengantryanlage wird für diese Bestrahlung mit einem beschränkten Winkelbereich nicht genutzt.

Deshalb sind in dem vorliegenden Ausführungsbeispiel einer Ionenstrahlanlage nach Fig. 1 zwei der drei Bestrahlungsräume mit einem sowohl preis- als auch gewichtsmäßig optimierten erfindungsgemäßen ersten Bestrahlungssystems 5 ausgestattet, das die material-, raum- und kostenaufwendigen Gantrylösungen vermeidet und mittels eines neuen asymmetrischen Scansystems einen zentralen Ionenstrahlablenkbereich von ±15° gegenüber der horizontalen Richtung gewährleistet.

Für die übrigen Bestrahlungen mit einem größeren erforderlichen Bestrahlungswinkelbereich und schneller reproduzierbarer Einstellung wird ein zweites Bestrahlungssystem bereitgestellt, das ebenfalls auf ein rotierendes Gantry verzichtet, wie es aus dem Stand der Technik bekannt ist, sofern eine technische Umsetzung der linearen Gantry möglich ist. Sonst kann der Bestrahlungsraum notfalls mit einer konventionellen RotationsGantry ausgestattet werden. Diese Einrichtung eines zweiten Bestrahlungssystems ist in dem Bestrahlungsraum 24 des Beispiels einer Ionenstrahlanlage nach Fig. 1 vorgesehen. Beide Strahlungssysteme, nämlich das asymmetrische Scansystem und das zweite Strahlungssystem mit größerem Ablenkwinkelbereich bieten den Vorteil der Kompaktheit in den notwendigen Gebäudeabmessungen sowie eine hohe mechanische Stabilität. Dadurch verringern sich beim Bau die Investitionskosten und im Betrieb werden zusätzliche Kontrollen und Nachjustierungen, wie sie beim Gantrysystem erforderlich sind, überflüssig.

Der Patientendurchsatz wird dann durch die Zuführung und die Positionierungskontrolle der Patienten auf der horizontalen Patientenliege bedingt. Um diesen Patientendurchsatz zu optimieren, ohne die Sicherheit zu beeinträchtigen, müssen die einzelnen Arbeitsabschnitte voneinander getrennt werden, damit sie von den jeweiligen Spezialisten ohne Zeitdruck und menschenwürdig durchgeführt werden können.

Ein Verfahren zur Durchführung der Bestrahlung in einer derartigen Anlage nach Fig. 1 umfaßt deshalb folgende Verfahrensschritte:
a) Immobilisieren des Patienten auf einer Patientenliege 1 in einem Vorbereitungsraum 12 der Ionenstrahlanlage 4,
b) Bestimmen des optimalen Bestrahlungswinkels für die Bestrahlungstherapie,
c) Auswahl des geeigneten ersten oder zweiten Bestrahlungssystems 5 oder 9,
d) Einstellen der Ablenkwinkel und der örtlichen Positionen der Ablenkvorrichtungen für den zentralen Ionenstrahlablenkbereich,
e) Einfahren, Einrasten und Einstellen der Patientenliege 1 relativ zu dem vorbestimmten optimalen Bestrahlungswinkel in dem jeweiligen Bestrahlungsraum 22, 23, 24,
f) Durchführen der Bestrahlung, eventuell mit Unterbrechungen für Positionsänderungen der Patientenliegen 1 und/oder der Ablenkwinkel der Ablenkvorrichtungen für eine Mehrfeldbestrahlung,
g) Rücktransfer des Patienten auf der Patientenliege 1 in einen Nachsorgeraum 37,
h) Entfernen der Immobilisierung des Patienten auf der Patientenliege 1.

Der Bedarf an Bestrahlungsräumen 22, 23, 24 ergibt sich aus den Patientenindikationen. Geht man von der Verteilung aus, die das bekannte mit drei Rotationsgantry bestückte Protonenbehandlungssystem der US-Patentschrift 4,870,287 umfaßt, so ergibt sich die Notwendigkeit von wenigstens zwei Bestrahlungsräumen mit asymmetrischem Scansystem gemäß dem ersten Bestrahlungssystem 5 und einem dritten Bestrahlungsraum 24 mit einem zweiten Bestrahlungssystem mit größerem Bestrahlungswinkel als es das erste Bestrahlungssystem ermöglicht. Um Engpässe zu vermeiden, sind die Vorbereitungsräume 12 der Ionenstrahlanlage gemäß Fig. 1 derart angeordnet, daß von jedem Vorbereitungsraum 12 jeder der Behandlungsräume 22, 23 und 24 erreicht werden kann. Nach den Erfahrungen mit den bisherigen Gantrysystemen benötigen etwa 20 bis 30 % der Tumorbehandlungsfälle eine Bestrahlung unter einem Bestrahlungswinkel, der größer als 15° ist. Somit sollte die Ionenstrahlanlage gemäß Fig. 1 den bisher anstehenden Bestrahlungsanforderungen vollkommen genügen, ohne daß eine einzige teure rotierende Gantryanlage erforderlich ist. Ferner ist in Fig. 1 deutlich zu sehen, daß die Bestrahlungsräume kompakt nebeneinanderliegen und äußerst kurze Strahlwege mit möglichst wenig Ablenkwinkeln mit den Beschleunigungseinrichtungen 28 und 29 als Strahlquelle verbunden sind.

Der Ionenstrahl 16 vom Beschleuniger wird über Klein-Winkelablenkungen 33, 35 und 36 als Strahlweichen auf drei Bestrahlungsräume 22, 23 und 24 geleitet, die sektorartig nebeneinanderliegen. Zwei benachbarte Bestrahlungsräume 22 und 23 sind jeweils mit dem ersten Bestrahlungssystem 5, einem asymmetrischen Scansystem, ausgestattet. Sie dienen der Bestrahlung im Winkelbereich von 0 bis 15° gegenüber der Horizontalen, d.h. für Prostata-Patienten sowie einem Großteil der Hirntumor- und Schädelbasistumor-Patienten. Der dritte Bestrahlungsraum 24 ist mit einer Ionenstrahlablenkvorrichtung für größere Bestrahlungswinkel gegenüber der Horizontalen zwischen 0 und 90° ausgestattet. Die Bestrahlungsräume 22, 23 und 34 werden von einem Kranz von Vorbereitungs- und Nachsorgeräumen 12 bzw. 37 umgeben, die über einen gemeinsamen Korridor 40 zwischen Bestrahlungsräumen 22, 23, 24 und Vorbereitungs- und Nachsorgeräumen 12 bzw. 37 erreicht werden. Diese erfindungsgemäße Ionenstrahlanlage kann durch weitere Bestrahlungssysteme bei Bedarf seitlich ergänzt werden, wie es die gestrichelten Ionenstrahlrichtungen 31 und 32 andeuten.

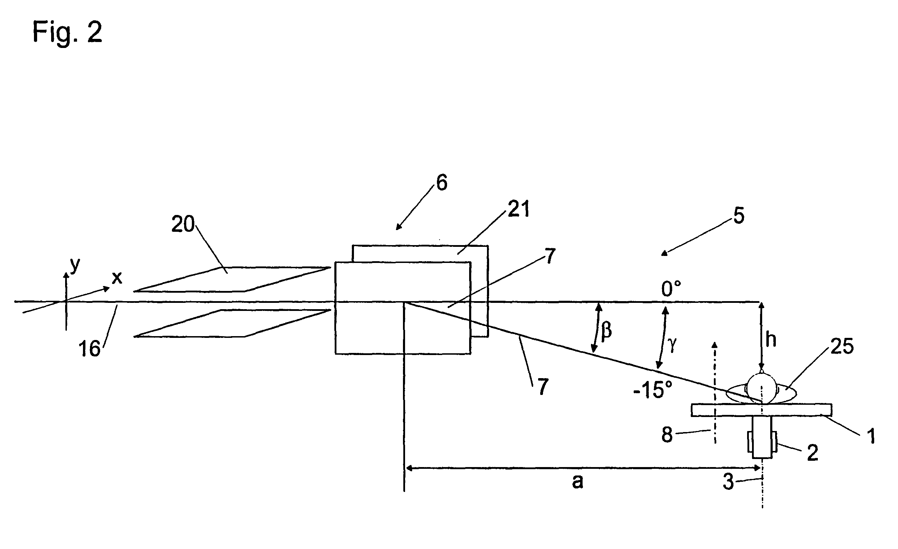

Fig. 2 zeigt eine Prinzipskizze eines ersten Bestrahlungssystems 5 mit asymmetrischem, örtlich feststehendem Scansystem, das im wesentlichen aus einem Ablenkmagneten 20 für ein horizontales Abtasten in X-Richtung einer zum zentralen Ionenstrahlablenkbereich 7 liegenden orthogonalen Ebene besteht. Von dem Ablenkmagneten 20 sind lediglich prinzipiell die Wirkflächen zwischen zwei Magnetschuhen abgebildet. Die Ablenkung in X-Richtung ist im Vergleich zu der gezeigten Ablenkung von -15° des zentralen Ionenstrahlablenkbereiches 7 relativ gering, denn die Ablenkung des asymmetrischen Scansystem soll in X-Richtung lediglich die Breite des Tumorvolumens abtasten. Hingegen ist der Ablenkmagnet 21, der in Y-Richtung wirkt, wesentlich größer ausgelegt, um einen zentralen Ionenstrahlablenkbereich bis zu -15° in Y-Richtung gegenüber der Horizontalen von 0° zu bewirken. Diesem zentralen Ionenstrahlablenkbereich 7 ist ein nicht gezeigter Scanbereich in Y-Richtung beim Bestrahlen des Tumorvolumens überlagert. Auch von dem Ablenkmagnet 21 sind lediglich die Wirkflächen zwischen zwei Polschuhen gezeigt. Dieses asymmetrische Scansystem erzeugt folglich relativ kleine Bestrahlungswinkel γ von bis zu ±15°, dabei wird der Strahl in ausreichender Höhe über der Patientenliege 1 geführt und durch Aktivieren eines vertikalen Ablenkmagneten 21, der Bestandteil des asymmetrischen Scansystems ist, in diesem Ausführungsbeispiel der Fig. 2 nach unten gelenkt. Die Bestrahlung des Patienten 25 erfolgt dann auf einer Patientenliege 2 mit variabler Höhe h, die je nach erforderlichem Bestrahlungswinkel γ justiert wird.

Bei einem Abstand von 5 m zwischen dem vertikalen Scanmagneten 21 und dem Patientenisozentrum, in dem sich das Tomovolumen befindet, ergeben sich die Höheneinstellungen der Tabelle 1.

**Tabelle 1**

| **"Gantry"-Winkel** | **Patientenhöhe** | **effektive Länge** |
|---|---|---|
| 0° | 0 cm | 5 m |
| + 5° | -45 cm | 5,02 m |
| + 10° | -90 cm | 5,08 m |
| + 15° | -135 cm | 5,18 m |

Eine Höhenanpassung der Patientenliege von 1,35 m kann entweder durch eine Verstellung der Patientenliege allein oder durch eine Höhenverstellung des Bodens im gesamten Bestrahlungsraum mit Patientenliege 1 oder durch Hubvorrichtungen sowohl für die Patientenliege 1 als auch für den Boden erfolgen.

Bei einer Winkeländerung für den zentralen Ionenstrahlablenkbereich mit Hilfe des vertikalen Ablenkmagneten 21 tritt ebenfalls eine Änderung der effektiven Länge zwischen Ablenkmagnet 21 und Isozentrum ein, die jedoch mit maximal 20 cm, wie es aus Tabelle 1 zu sehen ist, relativ klein bleibt, und in den Strahleinstellungen sowie der Bestrahlungsplanung berücksichtigt werden kann. Insgesamt ist mit Hilfe des Ablenkmagneten 21 für die vertikale Richtung ein Gesamtbereich von 30° möglich, nämlich von -15° bis +15°. Dazu muß lediglich die Hubvorrichtung 8 der Patientenliege 1 mit einem ausreichenden Hubweg von bis zu 2,7 m in dieser Ausführungsform ausgestattet sein. Außer der Höhenverstellung der Patientenliege mit Hilfe der Hubvorrichtung 8 gibt es bei diesem asymmetrischen Scansystem keine mechanisch veränderbaren Komponenten des Strahlführungssystem. Um die nötige Flexibilität des Strahls zu gewährleisten, wird das Strahlrohr als Sektor mit der nötigen Breite von ca. 20 cm und der nötigen Höhe von 1,35 m bei 15° Ablenkwinkel bei diesem Beispiel der Fig. 2 ausgeführt und mit einem entsprechenden Fenster von 20 cm x 1,35 cm verschlossen, das aus einem textilen Stützgewebe mit aufliegender Dichtefolie besteht. Die Detektoren des Kontrollsystems, wie Ionisations- und Vieldrahtkammer zur Intensitäts- und Ortsmessung können über die ganze Fensterfläche ausgedehnt werden, womit eine von mechanischen Einflüssen unabhängige Kontrollmessung der Ortslage und Intensität des Ionenstrahls in diesem ersten Bestrahlungssystem möglich wird.

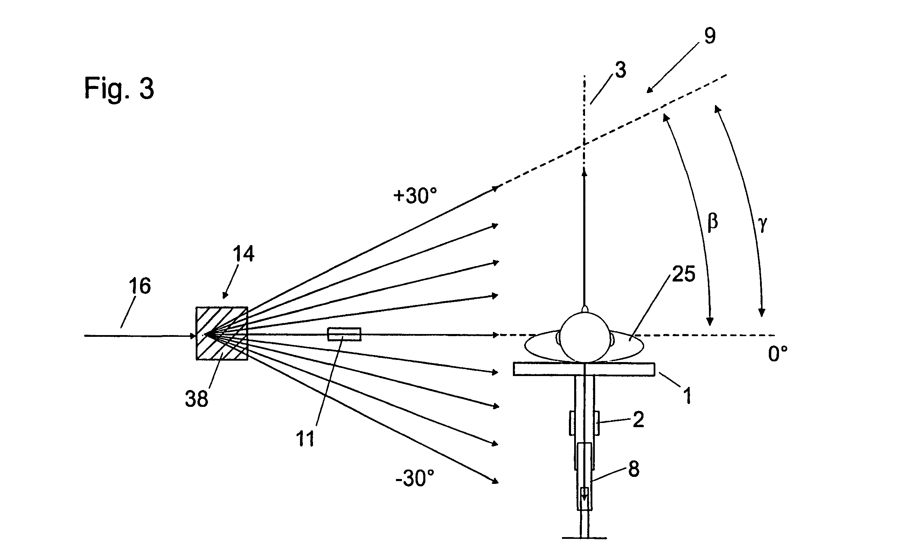

Fig. 3 zeigt eine Prinzipskizze einer Ausführungsform eines zweiten Bestrahlungssystems 9 mit ortsfester Ionenstrahlablenkvorrichtung 14 und vertikal verschiebbarer Patientenliege 1. Bei Winkeln über 15°, die durch das erste Bestrahlungssystem abgedeckt werden können und zwei Drittel bis drei Viertel der Bestrahlungsfälle abdeckt, sind ohne ein aufwendiges rotierendes Gantry nach Fig. 3 im Prinzip Bestrahlungswinkel von -30° bis +30° möglich, ohne daß das Bestrahlungssystem selbst bewegt wird, wie es bei dem rotierenden Gantry der Fall ist. Es wird lediglich der Erregerstrom des Ablenkmagneten 38, der zur Ionenstrahlablenkvorrichtung 14 gehört, gesteuert. Die Scanvorrichtung 11 selbst kann symmetrisch ausgeführt seinwird, d.h. die Ablenkung sowohl in X- als auch in Y-Richtung orthogonal zum zentralen Ionenstrahlablenkbereich kann in beiden Richtungen gleiche Größenordnungen aufweisen. In dem Beispiel der Fig. 3 ist die Hubvorrichtung 8 für die Patientenliege gerade soweit ausgefahren, daß das Isozentrum bei 0° liegt. Mit der Änderung des Bestrahlungswinkels im Bereich von -30° bis +30° wird bei dieser Ausführungsform nicht nur der Patient durch eine Höhenverstellung der Hubvorrichtung 8 der Patientenliege 1 bewegt, sondern es wird die symmetrische Scanvorrichtung 11 mit der Änderung des Bestrahlungswinkels γ geschwenkt.

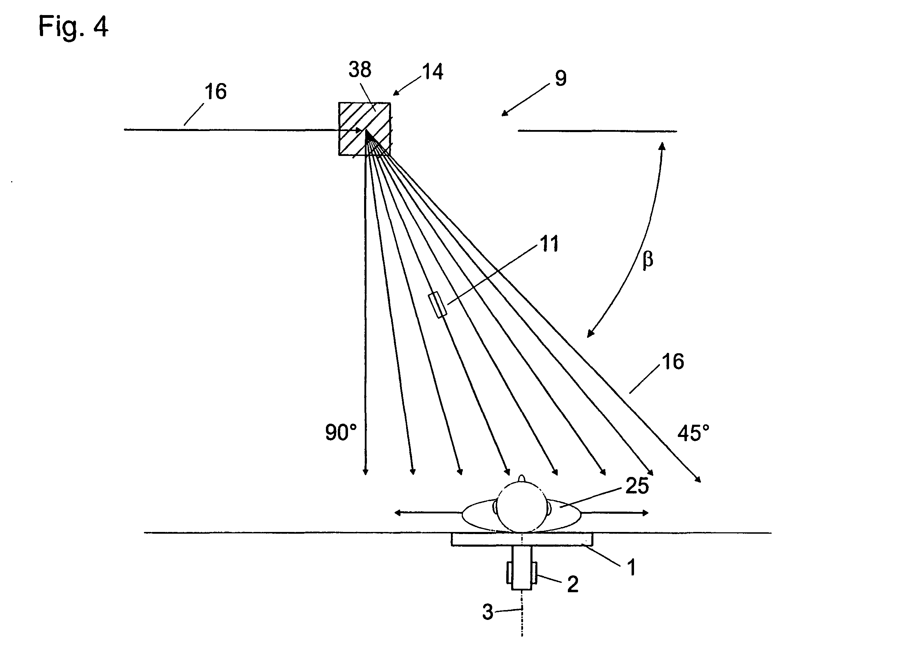

Fig. 4 zeigt eine Prinzipskizze einer weiteren Ausführungsform eines zweiten Bestrahlungssystems mit ortsfester Ionenstrahlablenkvorrichtung 14 und horizontal verschiebbarer Patientenliege 1. Im Unterschied zu Fig. 3 wird bei dieser Ausführungsform des zweiten Bestrahlungssystems 9 die Patientenliege 1 nicht vertikal, sondern horizontal in die Bestrahlungsposition verschoben. Dafür ist die ortsfeste Ionenstrahlablenkvorrichtung 14 oberhalb des Patienten angeordnet und bewirkt mit dem Ablenkmagneten 38 eine Ablenkung des Ionenstrahls 16 aus einer horizontalen Quelle, um den Ablenkwinkel β von 45° bis 90° zur Horizontalen bereitzustellen. Mit dem Verschieben der Patientenliege 1 muß auch gleichzeitig die symmetrische Scanvorrichtung 11 synchron geschwenkt werden, um das Tumorvolumen des Patienten 25 voll abtasten zu können.

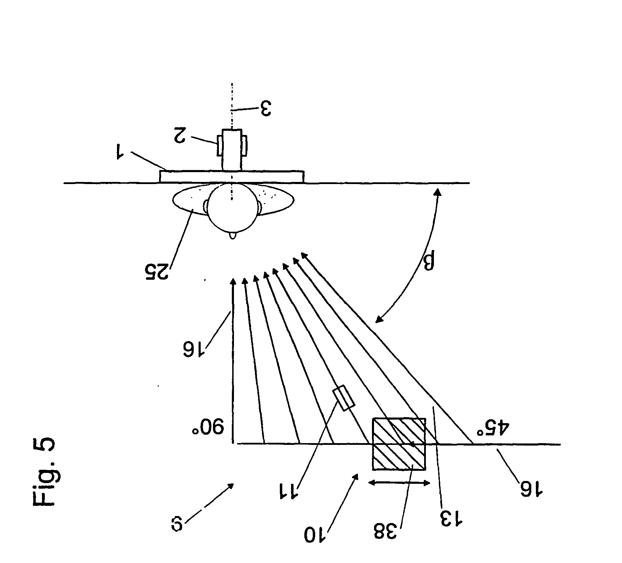

Fig. 5 zeigt eine Prinzipskizze einer weiteren Ausführungsform eines zweiten Bestrahlungssystems 9 mit ortsfester drehbarer Patientenliege 1 und einer auf einer horizontalen linearen Bahn 13 verschiebbaren Ionenstrahlablenkvorrichtung 10. In diesem Fall, bei dem die horizontale lineare Bahn 13 zur Verschiebung der Ionenstrahlablenkvorrichtung 10 oberhalb des Patienten angeordnet ist, kann ein Ablenkwinkel β zur horizontalen Ausrichtung von 45° bis 90° erreicht werden, ohne daß große Massen zu bewegen sind, da die Apertur des Ablenkmagneten 38 äußerst gering ausgeführt werden kann, zumal die symmetrische Scaneinheit 11 diesem Ablenkmagneten nachgeordnet ist. Auch in dieser Ausführungsform muß neben der Änderung des Erregerstroms im Ablenkmagneten 38 und seiner Bewegung auf der horizontalen linearen Bahn 13 die symmetrische Scaneinheit 11 entsprechend dem Ablenkwinkel β geschwenkt werden. Der Vorteil dieser Ausführungsform ist, daß die Patientenliege 1 vollständig ortsfest ausgeführt sein kann und lediglich zur Erweiterung des Bereichs des Bestrahlungswinkels γ drehbar um die vertikale Achse 3 ausgeführt wird.

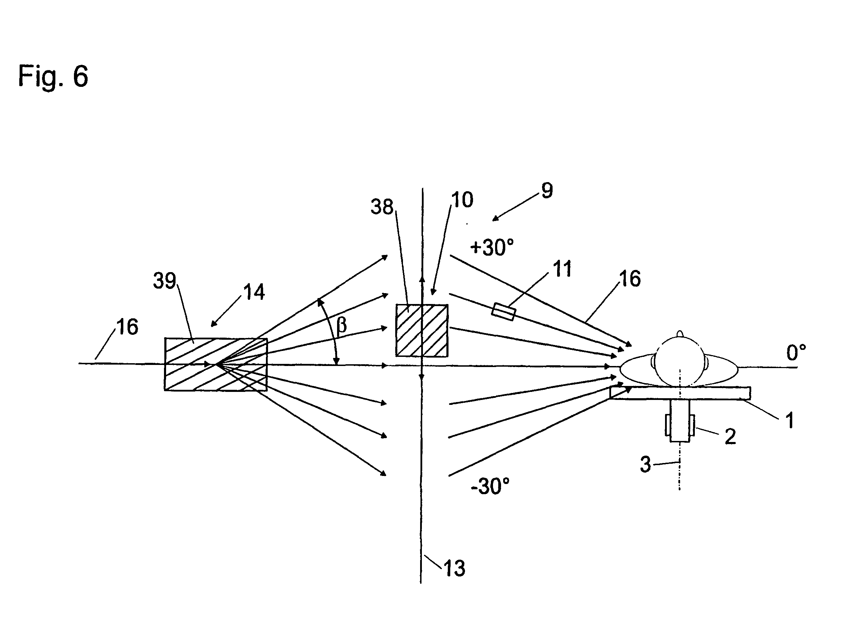

Fig. 6 zeigt eine Prinzipskizze einer weiteren Ausführungsform eines zweiten Bestrahlungssystems 9 mit ortsfester drehbarer Patientenliege 1 und auf einer vertikalen linearen Bahn 13 verschiebbaren Ionenstrahlablenkvorrichtung 10. Da die Ionenstrahlquelle einen horizontalen Ionenstrahl 16 liefert, lenkt zunächst bei einem Bereitstellen einer auf einer vertikalen linearen Bahn verschiebbaren Ionenstrahlablenkvorrichtung ein feststehender Ablenkmagnet 39 den Ionenstrahl aus der Horizontalen um den Ablenkwinkel β ab, und ein in vertikaler linearer Richtung verschiebbarer Ablenkmagnet 38 kann dann den Ionenstrahl auf die ortsfeste Patientenliege 1 richten. Diese Ausführungsform nach Fig. 6 hat den Vorteil, daß wiederum lediglich eine Hubvorrichtung, und zwar für die vertikal verschiebbare Ionenstrahlablenkvorrichtung 10 erforderlich ist, wobei eine derartige Hubvorrichtung mittels hydraulischer Mechaniken relativ preiswert und raumsparend durchgeführt werden kann. Mit der Hubvorrichtung muß auch in diesem Fall eine symmetrische Scaneinheit 11 mitgeführt werden, die gleichzeitig mit dem Ablenkwinkel β schwenkbar ist.

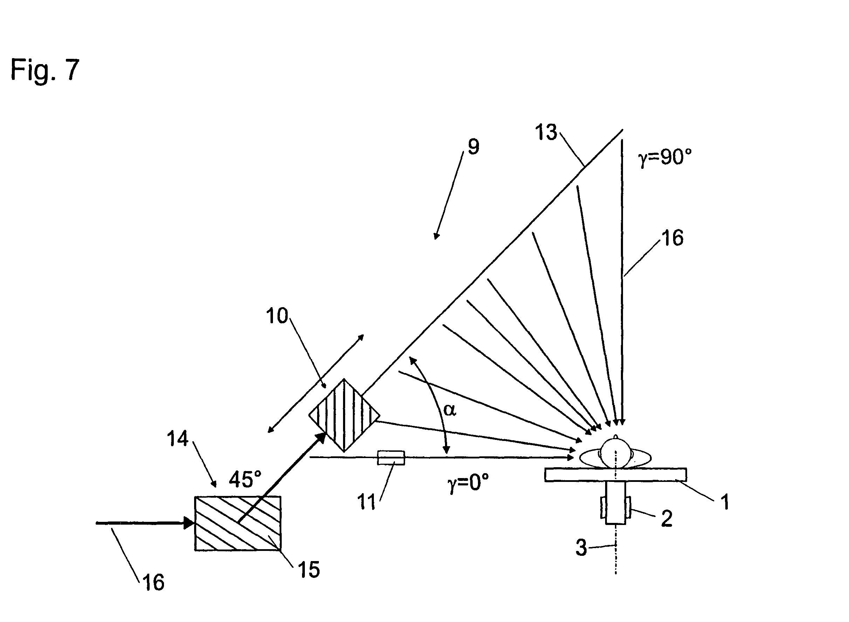

Fig. 7 zeigt eine Prinzipskizze einer weiteren Ausführungsform eines zweiten Bestrahlungssystems 9 mit ortfester drehbarer Patientenliege 1 und einer verschiebbaren Ionenstrahlablenkvorrichtung 10 auf einer schiefen linearen Bahn 13. Eine derartige schiefe lineare Bahn 13 für eine Ionenstrahlablenkvorrichtung 10 hat den Vorteil, daß die Winkeleinschränkungen, die noch für die Ausführungsbeispiele in den Fig. 3 bis Fig. 6 zu sehen waren, nun aufgehoben sind, indem mit dieser Ausführungsform Bestrahlungswinkel γ beginnend mit der Horizontalen 0° bis zu 90° verwirklicht werden können, ohne daß aufwendige rotierende Gantrysysteme zum Einsatz kommen. Die Ausführungsform nach Fig. 7 geht davon aus, daß der Ionenstrahl 16 in horizontaler Richtung unterhalb der Position der Patientenliege 1 in den Bestrahlungsraum eintritt. Ein erster Umlenkmagnet 15 lenkt den Ionenstrahl 16 in Richtung auf die schiefe lineare Bahn 13 ab und wird von der auf der schiefen Ebene 13 bewegbaren Ionenstrahlablenkvorrichtung 10 durch Variation des Erregerstroms des Elektromagneten in unterschiedlichen Winkeln so abgelenkt, daß jeweils die ortsfeste drehbare Patientenliege 1 im Isozentrum getroffen wird. Auch hier wird eine symmetrische Scaneinheit 11 mit der verschiebbaren Ionenstrahlablenkvorrichtung 10 mitgeführt und jeweils derart geschwenkt, daß sie in Richtung des Ionenstrahls ausgerichtet bleibt.

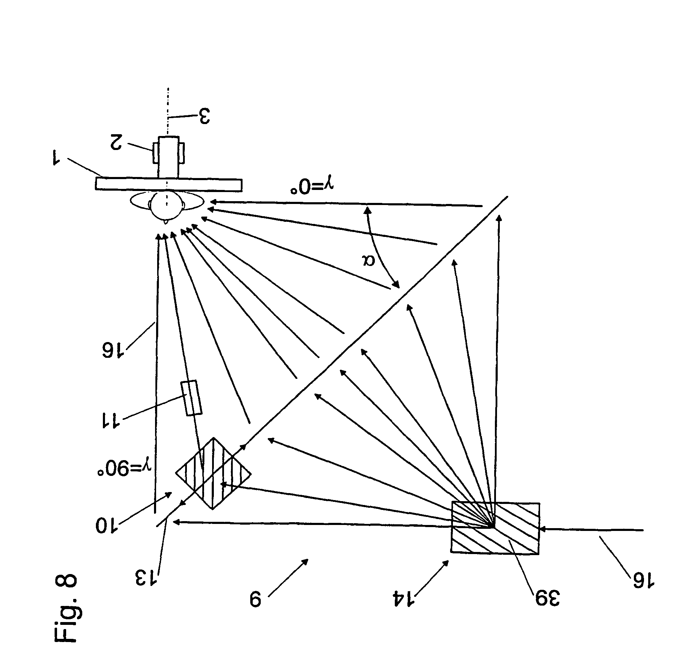

Fig. 8 zeigt eine Prinzipskizze einer weiteren Ausführungsform eines zweiten Bestrahlungssystems 9 mit ortsfester drehbarer Patientenliege 1 und einer Kombination aus ortfester und auf einer schiefen linearen Bahn verschiebbarer Ionenstrahlablenkvorrichtung. Dazu ist in dieser Ausführungsform angenommen, daß der Ionenstrahl 16 oberhalb der Patientenliege 1 dem Bestrahlungsraum zugeführt wird und zunächst auf einen ortsfesten Ablenkmagneten 39 trifft, der den Ionenstrahl zwischen 0° und 90° in bezug auf die horizontale Richtung ablenkt. Eine verschiebbare Ionenstrahlablenkvorrichtung 10 ist auf einer schiefen linearen Bahn 13 unter einem Winkel α bewegbar und lenkt den durch den ortsfesten Ablenkmagneten 39 abgelegten Ionenstrahl 16 auf die Patientenliege 1 zu. Auch bei dieser Ausführungsform wird eine symmetrische Scaneinheit 11 mit der verschiebbaren Ionenstrahlablenkvorrichtung 10 mitgeführt, um das gesamte Tumorvolumen im Isozentrum auf der Patientenliege 1 abzutasten.

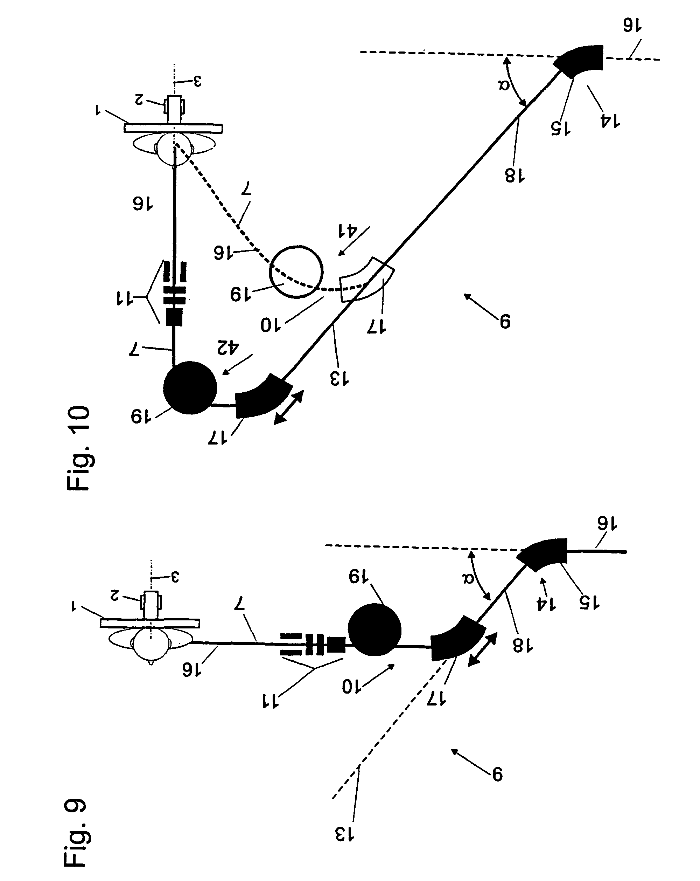

Fig. 9 zeigt eine Prinzipskizze einer weiteren Ausführungsform eines zweiten Bestrahlungssystems 9 mit ortsfester drehbarer Patientenliege 1 und einer unter einem Winkel von 45° gegenüber der horizontalen Richtung geneigten schiefen linearen Bahn 13 für eine verschiebbare Ionenstrahlablenkvorrichtung 10. Die Ionenstrahlablenkvorrichtung 10 besteht hier aus einem ortsfesten Umlenkmagneten, der dem in horizontaler Richtung in der Bestrahlungskammer ankommenden Ionenstrahl 16 in eine schiefe lineare Bahn, die zu der horizontalen Richtung um den Winkel α = 45° geneigt ist, umlenkt. Der umgelenkte Ionenstrahl 18 wird dann durch einen zweiten Umlenkmagneten 17 zunächst in eine horizontale Richtung konstant und unverändert umgelenkt. Ein nachgeordneter Solenoidmagnet 19 kann dann in Abhängigkeit von seinem Erregerstrom den Ionenstrahl von 0° bis 90° ablenken. Fig. 9 zeigt dazu die Situation, bei der der Solenoidmagnet 19 nicht aktiv ist und folglich der Ionenstrahl horizontal in Richtung auf die Patientenliege 1 geführt wird. Dem Solenoidmagneten 19 ist eine in dieser Position der Fig. 9 horizontal angeordnete symmetrische Scaneinheit 11 nachgeordnet, die durch entsprechende Ablenkfeld in X- und Y-Richtung orthogonal zum zentralen Ionenstrahlablenkbereich 7 das Tumorvolumen abscannen kann.

Fig. 10 zeigt eine Prinzipskizze der in Fig. 9 gezeigten Ausführungsform in zwei weiteren Positionen 41 und 42 der verschiebbaren Ionenstrahlablenkvorrichtung 10. Die mit einem gestrichelten Ionenstrahl 16 gekennzeichnete mittlere Position 41 zeigt die gleichbleibende Ablenkwirkung des zweiten Umlenkmagneten 17 und die erhöhte Umlenkwirkung des Solenoidmagneten 19, der in der zweiten Position 42 seine größte Wirkung mit einem um 90° abgewinkelten Ionenstrahl 16 darstellt. Die symmetrische Scaneinheit 11 wird dabei jeweils mit dem Ionenstrahl 16 mitgeschwenkt, so daß in allen Positionen des Bestrahlungwinkels γ das Tumorvolumen vollständig abgetastet werden kann.

Somit wird mit Hilfe dieses 45° linearen Gantrys ein aufwendiges Rotationsgantrysystem vermieden, und es genügt eine lineare Verschiebung des Magnetsystems auf einer schiefen Ebene, wie in diesem Beispiel mit einer 45°-Steigung. Der Strahl wird dabei zunächst von der horizontalen Ebene auf die 45°-Ebene gelenkt. Auf dieser Ebene muß ein Satz von beweglichen Magneten mit maximaler Ablenkkapazität von 135° installiert werden. Für eine Bestrahlung von 0° (horizontal) sitzt das bewegliche System an seinem untersten Punkt und lenkt durch Aktivierung eines 45°-Dipolmagneten den Strahl unter 0° auf den Patienten.

Bei einer vertikalen Bestrahlung (90°) muß der bewegliche Magnetsatz auf das obere Ende der 45° schiefen Ebene gefahren werden, und das zweite Magnetablenksystem, das durch in diesem Beispiel durch einen Solenoidmagneten dargestellt wird, zusätzlich auf 90° aktiviert werden. Alle Winkel zwischen 0° und 90° können somit durch Zwischenstellung und entsprechenden Magneterregungen realisiert werden. Bestrahlungen zwischen 90° und 180° werden durch einfache Drehung der Patientenliege ermöglicht.

Bei einer maximalen Höhe von 6 m über der Patientenliege 1 kann die Ionenstrahlscaneinheit 11 hinter dem letzten Dipolmagneten untergebracht werden. Dadurch können die Aperturen der Dipolmagnete kleingehalten werden und damit auch das Gewicht wesentlich gegenüber den Gantrylösungen verringert werden. Die wesentlichen Vorteile des in den Figuren 9 und 10 abgebildeten Systems mit einer schiefen Ebene wurden bereits im Detail beschrieben.

## Patentansprüche

1. Ionenstrahlanlage zur Bestrahlung von Tumorgewebe unter unterschiedlichen Bestrahlungswinkeln (γ) in Bezug auf eine horizontal angeordnete Patientenliege (1),
**dadurch gekennzeichnet, dass**
die Patientenliege (1) eine Drehvorrichtung (2) um eine vertikale Achse (3) und die Ionenstrahlanlage (4) mindestens eines der folgenden Bestrahlungisysteme aufweist:
- ein erstes Bestrahlungssystem (5) mit einem symmetrischen oder asymmetrischen örtlich feststehenden Scansystem (6) mit Ablenkmagneten, das einen zentralen Ionenstrahlablenkungsbereich (7) für Ablenkungswinkel (β) von bis zu ±15° gegenüber der horizontalen Richtung aufweist und eine Abtastung eines Tumorvolumens in dem zentralen Ionenstrahlablenkbereich (7) ermöglicht und eine zusätzliche Hubvorrichtung (8) für die Patientenliege (1) aufweist, wobei die Hubvorrichtung (8) und die Drehvorrichtung (2) der Patientenliege zur Anpassung an den Ablenkwinkel (β) einstellbar und bei der Bestrahlung unveränderbar ist,
- ein zweites Bestrahlungssystem (9), das eine linear verschiebbare und örtlich fixierbare Ionenstrahlablenkvorrichtung (10) für einen gegenüber dem ersten Bestrahlungssystem (5) größeren Bereich des Ablenkwinkels (β) und eine stromabwärts der Ionenstrahlablenkvorrichtung (10) angeordnete und synchron mit dem Ablenkwinkel (β) der Ionenstrahlablenkvorrichtung (10) schwenkbare symmetrische Scaneinheit (11) zur Abtastung des Tumorvolumens aufweist, wobei eine Drehvorrichtung (2) und eine Hubvorrichtung (8) der Patientenliege (1) zur Anpassung der Patientenliege (1) an den Ablenkwinkel (β) einstellbar und bei der Bestrahlung unveränderbar ist.

2. Ionenstrahlanlage nach Anspruch 1,
**dadurch gekennzeichnet, daß**
die Ionenstrahlanlage (4) mindestens zwei erste Bestrahlungssysteme (5) und ein zweites Bestrahlungssystem (9) aufweist.

3. Ionenstrahlanlage nach Anspruch 1 oder Anspruch 2,
**dadurch gekennzeichnet, daß**
die Ionenstrahlanlage mindestens einen Vorbereitungsraum (12) pro Bestrahlungssystem (5, 9) mit jeweils einer Patientenliege (1) aufweist.

4. Ionenstrahlanlage nach einem der vorhergehenden Ansprüche,
**dadurch gekennzeichnet, daß**
das zweite Bestrahlungssystem (9) eine mit variablem Ablenkwinkel (β) feststehende Ionenstrahlablenkvorrichtung (14) aufweist, die seitlich von der Patientenliege (1) angeordnet ist und die Patientenliege (1) eine zusätzliche vertikale Hubvorrichtung (8) aufweist.

5. Ionenstrahlanlage nach einem der Ansprüche 1 bis 3,
**dadurch gekennzeichnet, daß**
das zweite Bestrahlungssystem (9) eine mit variablem Ablenkwinkel (β) feststehende Ionenstrahlablenkvorrichtung (14) aufweist, die vertikal oberhalb der Patientenliege (1) angeordnet ist und die Patientenliege (1) in horizontaler Richtung verschiebbar ist.

6. Ionenstrahlanlage nach einem der Ansprüche 1 bis 3,
**dadurch gekennzeichnet, daß**
das zweite Bestrahlungssystem (9), eine auf einer horizontalen linearen Bahn (13) verschiebbare Ionenstrahlablenkvorrichtung (10) mit variablem Ablenkwinkel (β) aufweist, und die Patientenliege (1) lateral fixiert ist.

7. Ionenstrahlanlage nach einem der Ansprüche 1 bis 3,
**dadurch gekennzeichnet, daß**
das zweite Bestrahlungssystem (9) eine mit variablem Ablenkwinkel (β) feststehende Ionenstrahlablenkvorrichtung (14) und eine auf einer vertikalen linearen Bahn (13) verschiebbare Ionenstrahlablenkvorrichtung (10) mit variablem Ablenkwinkel (β) aufweist, und die Patientenliege (1) lateral fixiert ist.

8. Ionenstrahlanlage nach einem der Ansprüche 1 bis 3,
**dadurch gekennzeichnet, daß**
das zweite Bestrahlungssystem (9) eine auf einer schiefen linearen Bahn (13) gegenüber der horizontalen Richtung unter einem Winkel α linear verschiebbare Ionenstrahlablenkvorrichtung (10) für einen Ablenkwinkel (β) im Bereich von -α bis -(α + 90°) aufweist und die Patientenliege (1) lateral fixiert ist.

9. Ionenstrahlanlage nach einem der Ansprüche 1 bis 3,
**dadurch gekennzeichnet, daß**
das zweite Bestrahlungssystem (9) eine ortsfeste Ionenstrahlablenkvorrichtung (14) mit variablem Ablenkwinkel (β) und eine auf einer schiefen linearen Bahn (13) gegenüber der horizontalen Richtung unter einem Winkel α linear verschiebbare Ionenstrahlablenkvorrichtung (10) für einen Ablenkwinkel (β) im Bereich von -α bis -(α + 90°) aufweist und die Patientenliege (1) lateral fixiert ist.

10. Ionenstrahlanlage nach Anspruch 8 oder 9,
**dadurch gekennzeichnet, daß**
der Winkel α der schiefen linearen Bahn 45° aufweist.

11. Ionenstrahlanlage nach Anspruch 9 oder 10,
**dadurch gekennzeichnet, daß**
das zweite Bestrahlungssystem (9) einen ersten Umlenkmagneten (15) zum Umlenken eines hochbeschleunigten horizontalen Ionenstrahls (16) aufweist, der den Ionenstrahl (16) um den Ablenkwinkel α in die schiefe lineare Bahn (13) umlenkt und einen zweiten Umlenkmagneten (17) aufweist, der den abgelenkten Ionenstrahl (18) von der schiefen linearen Bahn (13), auf welcher der zweite Umlenkmagnet (17) verschiebbar ist, zurück in die horizontale Richtung lenkt und einen mit dem zweiten Umlenkmagneten (17) verschiebbaren Solenoidmagnet (19) zum Umlenken des Ionenstrahls (16) in Richtung auf die Patientenliege (1) in einem vorbestimmten Bestrahlungswinkel (8) aufweist.

12. Ionenstrahlanlage nach einem der vorhergehenden Ansprüche,
**dadurch gekennzeichnet, daß**
das erste Bestrahlungssystem (5) einen ersten Ablenkmagneten (20) zum Abtasten des Tumorvolumens in einer X-Richtung der orthogonalen Ebene zum zentralen Ionenstrahlablenkbereich (7) aufweist und einen Ablenkmagneten (21) zum Ablenken in die Richtung des zentralen Ionenstrahlablenkbereichs (7) in einer Y-Richtung der orthogonalen Ebene zum zentralen Ionenstrahlablenkbereich (7) mit zusätzlicher Überlagerung zur Abtastung des Tumorvolumens in dieser Richtung aufweist.

13. Ionenstrahlanlage nach einem der vorhergehenden Ansprüche,
**dadurch gekennzeichnet, daß**
der Abstand zwischen dem Ablenkmagneten (21) in Y-Richtung und der Patientenliege (1) des ersten Bestrahlungssystems (5) zwischen 5 und 7 Meter beträgt.

14. Ionenstrahlanlage nach einem der vorhergehenden Ansprüche,
**dadurch gekennzeichnet, daß**
der Abstand zwischen der verschiebbaren linearen Bahn (13) des Solenoidmagneten (19) und der Patientenliege (1) 3 bis 6 Meter beträgt und die mitverschiebbare magnetische Scaneinheit (11) in diesem Bereich angeordnet ist.

15. Ionenstrahlanlage nach einem der vorhergehenden Ansprüche,
**dadurch gekennzeichnet, daß**
der gesamte Boden des Bestrahlungsraumes (22, 23, 24) beim Anheben der Patientenliege (1) mit angehoben ist.

16. Ionenstrahlanlage nach einem der vorhergehenden Ansprüche,
**dadurch gekennzeichnet, daß**
die Ionenstrahlanlage (4) Ionisationskammermeßvorrichtungen und Vieldrahtkammermeßvorrichtungen zur Ortsmessung und Überwachung des abtastenden Ionenstrahls (16) aufweist.

17. Ionenstrahlanlage nach einem der vorhergehenden Ansprüche,
**dadurch gekennzeichnet, daß**
die Bestrahlungssysteme (5, 9) in Bestrahlungsräumen (22, 23, 24) die kompakt und kreissegmentförmig nebeneinander liegen, angeordnet sind.

18. Ionenstrahlanlage nach einem der vorhergehenden Ansprüche,
**dadurch gekennzeichnet, daß**
die Ionenstrahlanlage (4) eine Sichtkontrolle für die Patientenliege (1) im Bestrahlungsraum (22, 23, 24) aufweist, um definierte Drehwinkeländerungen zwischen unterschiedlichen Einstellungen für eine Mehrfeldbestrahlung der Patientenliege (1) zu überwachen.

19. Ionenstrahlanlage nach einem der vorhergehenden Ansprüche,
**dadurch gekennzeichnet, daß**
die Bestrahlungssysteme (5, 9) Einrastvorrichtungen zur Aufnahme und Positionierung einer Patientenliege (1) im Bestrahlungsraum (22, 23, 24) aufweisen.

## Claims

1. Ion beam system for irradiating tumour tissues at various irradiation angles (γ) in relation to a horizontally arranged patient couch (1),
**characterised in that**
the patient couch (1) has an apparatus (2) for rotation about a vertical axis (3), and the ion beam system (4) has at least one of the following irradiation systems:
- a first irradiation system (5) having a symmetrical or asymmetrical scanning system with deflection magnets (6) of fixed location, which has a central ion beam deflection region (7) for deflection angles (β) of up to ±15° with respect to the horizontal direction and makes possible scanning of a tumour volume in the central ion beam deflection region (7), and which has an additional lifting apparatus (8) for the patient couch (1), the lifting apparatus (8) and the apparatus (2) for rotation of the patient couch being adjustable for adaptation to the deflection angle (β) and being non-modifiable during irradiation;
- a second irradiation system (9), which has an ion beam deflection apparatus (10), linearly displaceable and arranged to be fixed in terms of location, for a deflection angle (β) range greater than the first irradiation system (5) and a symmetrical scanning unit (11) for scanning the tumour volume, which scanning unit (11) is arranged downstream of the ion beam deflection apparatus (10) and is arranged to pivot synchronously with the deflection angle (β) of the ion beam deflection apparatus (10), an apparatus (2) for rotation of the patient couch (1) and a lifting apparatus (8) for the patient couch (1) being adjustable for adaptation of the patient couch (1) to the deflection angle (β) and being non-modifiable during irradiation.

2. Ion beam system according to claim 1,
**characterised in that**
the ion beam system (4) has at least two first irradiation systems (5) and a second irradiation system (9).

3. Ion beam system according to claim 1 or claim 2,
**characterised in that**
the ion beam system has at least one preparation room (12) per irradiation system (5, 9), each having a patient couch (1).

4. Ion beam system according to one of the preceding claims,
**characterised in that**
the second irradiation system (9) has an ion beam deflection apparatus (14) of fixed location and of variable deflection angle (β) arranged to the side of the patient couch (1), and the patient couch (1) has an additional vertical lifting apparatus (8).

5. Ion beam system according to one of claims 1 to 3,
**characterised in that**
the second irradiation system (9) has an ion beam deflection apparatus (14) of fixed location and of variable deflection angle (β) arranged vertically above the patient couch (1), and the patient couch (1) is displaceable in a horizontal direction.

6. Ion beam system according to one of claims 1 to 3,
**characterised in that**
the second irradiation system (9) has an ion beam deflection apparatus (10) of variable deflection angle (β) that is displaceable on a horizontal linear track (13), and the patient couch (1) is fixed in position laterally.

7. Ion beam system according to one of claims 1 to 3,
**characterised in that**
the second irradiation system (9) has an ion beam deflection apparatus (14) of fixed location and of variable deflection angle (β) and an ion beam deflection apparatus (10) of variable deflection angle (β) that is displaceable on a vertical linear track (13), and the patient couch (1) is fixed in position laterally.

8. Ion beam system according to one of claims 1 to 3,
**characterised in that**
the second irradiation system (9) has an ion beam deflection apparatus (10), linearly displaceable on a sloping linear track (13) at an angle α with respect to the horizontal direction, for a deflection angle (β) in the range from -α to - (α + 90°), and the patient couch (1) is fixed in position laterally.

9. Ion beam system according to one of claims 1 to 3,
**characterised in that**
the second irradiation system (9) has an ion beam deflection apparatus (14) of fixed location and of variable deflection angle (β) and has an ion beam deflection apparatus (10), linearly displaceable on a sloping linear track (13) at an angle α with respect to the horizontal direction, for a deflection angle (β) in the range from -α to - (α + 90°), and the patient couch (1) is fixed in position laterally.

10. Ion beam system according to claim 8 or 9,
**characterised in that**
the angle α of the sloping linear track is 45°.

11. Ion beam system according to claim 9 or 10,
**characterised in that**
the second irradiation system (9) has a first deflection magnet (15) for deflecting a highly accelerated horizontal ion beam (16), which deflects the ion beam (16), through the deflection angle α, to the sloping linear track (13), and has a second deflection magnet (17), which directs the deflected ion beam (18) from the sloping linear track (13), on which the second deflection magnet (17) is displaceable, back into the horizontal direction, and has a solenoid magnet (19), which is displaceable together with the second deflection magnet (17), for deflecting the ion beam (16) towards the patient couch (1) at a predetermined irradiation angle (8).

12. Ion beam system according to one of the preceding claims,
**characterised in that**
the first irradiation system (5) has a first deflection magnet (20) for scanning the tumour volume in an X direction of the plane orthogonal to the central ion beam deflection region (7) and has a deflection magnet (21) for deflecting in the direction of the central ion beam deflection region (7) in a Y direction of the plane orthogonal to the central ion beam deflection region (7) with additional superimposition for scanning the tumour volume **in that** direction.

13. Ion beam system according to one of the preceding claims,
**characterised in that**
the spacing between the deflection magnet (21) in the Y direction and the patient couch (1) of the first irradiation system (5) is between 5 and 7 metres.

14. Ion beam system according to one of the preceding claims,
**characterised in that**
the spacing between the displaceable linear track (13) of the solenoid magnet (19) and the patient couch (1) is from 3 to 6 metres and the co-displaceable magnetic scanning unit (11) is arranged **in that** region.

15. Ion beam system according to one of the preceding claims,
**characterised in that**
the entire floor of the irradiation room (22, 23, 24) is also raised when the patient couch (1) is raised.

16. Ion beam system according to one of the preceding claims,
**characterised in that**
the ion beam system (4) has ionisation chamber measurement apparatuses and multi-wire chamber measurement apparatuses for measuring the location of, and monitoring, the scanning ion beam (16).

17. Ion beam system according to one of the preceding claims,
**characterised in that**
the irradiation systems (5, 9) are arranged in irradiation rooms (22, 23, 24) that are located next to one another compactly and in the shape of segments of a circle.

18. Ion beam system according to one of the preceding claims,
**characterised in that**
the ion beam system (4) has a visual checking means for the patient couch (1) in the irradiation room (22, 23, 24) in order to monitor defined angle-of-rotation changes between various settings for multi-field irradiation of the patient couch (1).

19. Ion beam system according to one of the preceding claims,
**characterised in that**
the irradiation systems (5, 9) have locking apparatuses for taking up and positioning a patient couch (1) in the irradiation room (22, 23, 24).

## Revendications

1. Installation à faisceau ionique pour irradier des tissus tumoraux sous divers angles d'exposition (γ) par rapport à une table d'examen (1) disposée horizontalement, **caractérisée en ce que** la table d'examen (1) présente un dispositif tournant (2) autour d'un axe vertical (3) et l'installation à faisceau ionique (4) présentant au moins l'un des systèmes d'irradiation suivant :
- un premier système d'irradiation (5) avec un système à balayage symétrique ou asymétrique localement fixe (6) doté d'aimants de déviation, qui présente une zone centrale de déviation du faisceau ionique (7) pour des angles de déviation (β) allant jusqu'à ± 15° par rapport à la direction horizontale et qui permet une analyse du volume d'une tumeur dans la zone centrale (7)de déviation du faisceau ionique, et un dispositif de levage (8) supplémentaire pour la table d'examen (1), le dispositif de levage (8) et le dispositif tournant (2) de la table d'examen (8) étant réglables pour ajuster l'angle de déviation (β) et étant non modifiables lors de l'irradiation,
- un deuxième système d'irradiation (9), qui présente un dispositif de déviation du faisceau ionique (10) pouvant être décalé de manière linéaire et fixé localement pour une zone plus importante de l'angle de déviation (β) par rapport au premier système d'irradiation (5), et une unité (11) de balayage symétrique placée en aval du dispositif de déviation du faisceau ionique (10) et pouvant être basculée en synchronisation avec l'angle de déviation (β) du dispositif de déviation du faisceau ionique (10) pour analyse du volume tumoral, un dispositif tournant (2) et un dispositif de levage (8) de la table d'examen (1) étant réglables pour ajuster la table d'examen (1) à l'angle de déviation (β) et n'étant pas modifiables lors de l'irradiation.

2. Installation à faisceau ionique selon la revendication 1, **caractérisée en ce que** l'installation à faisceau ionique (4) présente au moins deux premiers systèmes d'irradiation (5) et un deuxième système d'irradiation (9).

3. Installation à faisceau ionique selon la revendication 1 ou la revendication 2, **caractérisée en ce que** l'installation à faisceau ionique présente au moins un espace de préparation (12) par système d'irradiation (5, 9) doté respectivement d'une table d'examen (1).

4. Installation à faisceau ionique selon l'une quelconque des revendications précédentes, **caractérisée en ce que** le deuxième système d'irradiation (9) présente un dispositif (14) de déviation du faisceau ionique fixe doté d'un angle de déviation variable (β) qui est placé latéralement par rapport à la table d'examen (1) et la table d'examen (1) présentant un dispositif de levage vertical (8) supplémentaire.

5. Installation à faisceau ionique selon l'une quelconque des revendications 1 à 3, **caractérisée en ce que** le deuxième système d'irradiation (9) présente un dispositif (14) de déviation du faisceau ionique fixe, doté d'un angle de déviation (β) variable, qui est placé verticalement au-dessus de la table d'examen (1), et la table d'examen (1) peut être décalée en direction horizontale.

6. Installation à faisceau ionique selon l'une quelconque des revendications 1 à 3, **caractérisée en ce que** le deuxième système d'irradiation (9) présente un dispositif de déviation du faisceau ionique (10) pouvant être décalé sur une piste linéaire horizontale (13), doté d'un angle de déviation (β) variable, et la table d'examen (1) est fixée latéralement.

7. Installation à faisceau ionique selon l'une quelconque des revendications 1 à 3, **caractérisée en ce que** le deuxième système d'irradiation (9) présente un dispositif de déviation du faisceau ionique (14) fixe avec un angle de déviation variable (β) et un dispositif de déviation du faisceau ionique (10) doté d'un angle de déviation (β) variable, pouvant être décalé sur une piste linéaire verticale (13) et la table d'examen (1) est fixée latéralement.

8. Installation à faisceau ionique selon l'une quelconque des revendications 1 à 3, **caractérisée en ce que** le deuxième système d'irradiation (9) présente un dispositif de déviation du faisceau ionique (10) pouvant être décalé de manière linéaire sur une piste linéaire oblique (13) par rapport à la direction horizontale sous un angle α, pour un angle de déviation β dans la plage de -α à -(α + 90°) et la table d'examen (1) étant fixée latéralement.

9. Installation à faisceau ionique selon l'une quelconque des revendications 1 à 3, **caractérisée en ce que** le deuxième système d'irradiation (9) présente un dispositif de déviation du faisceau ionique localement fixe (14) avec un angle de déviation variable (β) et un dispositif de déviation du faisceau ionique (10) pouvant être décalé de manière linéaire sur une piste linéaire oblique (13) par rapport à la direction horizontale sous un angle α, pour un angle de déviation (β) dans la plage de -α à -(α + 90°) et la table d'examen (1) étant fixée latéralement.

10. Installation à faisceau ionique selon l'une quelconque des revendications 8 ou 9, **caractérisée en ce que** l'angle α est à 45° C de la piste linéaire oblique.

11. Installation à faisceau ionique selon l'une quelconque des revendications 9 ou 10, **caractérisée en ce que** le deuxième système d'irradiation (9) présente un premier aimant de déviation (15) pour dévier un faisceau ionique horizontal à vitesse élevée (16), qui dévie le faisceau ionique (16) de l'angle de déviation α dans la piste linéaire oblique (13), et un deuxième aimant de déviation (17) qui redirige le faisceau ionique dévié (18) dans la direction horizontale depuis la piste linéaire oblique (13) sur laquelle le deuxième aimant de déviation (17) peut être déplacé, et un aimant solénoïde (19) pouvant être décalé avec le deuxième aimant de déviation (17) pour dévier le faisceau ionique (16) en direction de la table d'examen (1) dans un angle d'irradiation prédéterminé (8).

12. Installation à faisceau ionique selon l'une quelconque des revendications précédentes, **caractérisée en ce que** le premier système d'irradiation (5) présente un premier aimant de déviation (20) pour analyser le volume tumoral dans une direction X du plan orthogonal par rapport à la zone centrale (7) de déviation du faisceau ionique et un aimant de déviation (21) pour dévier en direction de la zone centrale (7) de déviation du faisceau ionique dans une direction Y du plan orthogonal par rapport à la zone centrale (7) de déviation du faisceau ionique avec superposition supplémentaire pour analyser le volume tumoral dans cette direction.

13. Installation à faisceau ionique selon l'une quelconque des revendications précédentes, **caractérisée en ce que** la distance entre l'aimant de déviation (21) dans la direction Y et la table d'examen (1) du premier système d'irradiation (5) est de 5 à 7 mètres.

14. Installation à faisceau ionique selon l'une quelconque des revendications précédentes, **caractérisée en ce que** la distance entre la piste linéaire pouvant être décalée (13) de l'aimant solénoïde (19) et de la table d'examen (1) est de 3 à 6 mètres et l'unité de balayage magnétique pouvant être décalée en même temps (11) est placée dans ce domaine.

15. Installation à faisceau ionique selon l'une quelconque des revendications précédentes, **caractérisée en ce que** le plancher total de l'espace d'irradiation (22, 23, 24) est également élevé lors du levage de la table d'examen (1).

16. Installation à faisceau ionique selon l'une quelconque des revendications précédentes, **caractérisée en ce que** l'installation à faisceau ionique (4) présente des dispositifs de mesure de la chambre d'ionisation et des dispositifs de mesure de la chambre multifibre pour mesure locale et surveillance du faisceau ionique exerçant le balayage (16).

17. Installation à faisceau ionique selon l'une quelconque des revendications précédentes, **caractérisée en ce que** les systèmes de rayonnement (5, 9) dans des espaces de rayonnement (22, 23, 24) sont placés de manière compacte et en forme de cercle les uns par rapport aux autres.

18. Installation à faisceau ionique selon l'une quelconque des revendications précédentes, **caractérisée en ce que** l'installation à faisceau ionique (4) présente un contrôle visuel pour la table d'examen (1) dans l'espace d'irradiation (22, 23, 24), pour surveiller des modifications d'angle de rotation définies entre des positionnement différents pour une irradiation à champs multiples de la table d'examen (1).

19. Installation à faisceau ionique selon l'une quelconque des revendications précédentes, **caractérisée en ce que** les systèmes d'irradiation (5, 9) présentent des dispositifs d'arrêt pour la réception et le positionnement d'une table d'examen (1) dans l'espace d'irradiation (22, 23, 24).
